# EUROPEAN PATENT SPECIFICATION

(11) **EP 1 545 321 B1**
(45) Date of publication and mention of the grant of the patent: **17.03.2010**
(21) Application number: 03764469.7
(22) Date of filing: 11.07.2003
(51) Int. Cl.: A61B 17/00

(54) **PERCUTANEOUS DEVICE FOR HARVESTING TUBULAR BODY MEMBERS**
PERKUTANE VORRICHTUNG ZUM ENTNEHMEN VON RÖHRENFÖRMIGEN KÖRPERGLIEDERN
DISPOSITIF PERCUTANE POUR SAISIR DES PARTIES TUBULAIRES DU CORPS

(30) Priority: 11.07.2002 US 395248 P; 24.05.2003 US 444773
(43) Date of publication of application: 29.06.2005
(73) Proprietor: Scottsdale Medical Devices, Inc., Scottsdale, AZ 85260 (US)
(72) Inventor: OPIE, John, C., Scottsdale, AZ 85258 (US); JOYCE, Stephen J., Joyce, Phoenix, AZ 85013 (US)
(74) Representative: Lawrence, John
(86) International application number: PCT/US2003/021651
(87) International publication number: WO 2004/006777

(56) References cited:
- DE-A- 19 754 779
- DE-A- 19 817 979
- GB-A- 2 082 459
- US-A- 6 022 313
- US-B1- 6 193 653

## Description

### Field of the Invention

The present invention relates to devices for removing tubular body members, particularly blood vessels, from the body of a human or animal.

### Background of the Invention

Various tubular structures in the body ("tubular body members") are sometimes removed, either for somewhere else in the body or simply because removal is desired or necessary. As used herein, the terms "harvest," "dissect" and "remove," when used in connection with the removal of a tubular body member from the body, are synonymous. Tabular body members include blood vessels, such as arteries and veins, tendons, bile ducts and other structures. For example, the long sapheneous vein (LSV) located in subcutaneous fatty tissue in an anteromedial compartment of the lower leg and thigh is sometimes removed for use in (1) arterial bypass surgery, including coronary artery bypass, and peripheral arterial surgery bypasses, and (2) preparing an arteriovenus (AV) loop for dialysis. The length of the harvested LSV may vary from 20 cm to 100 cm.

Traditionally, the LSV has been removed by making a long incision along the leg from about the ankle to the groin, or by making a series of multiple, bridged incisions. Tissue (primarily fat) including the LSV is dissected from the leg through the incision(s) and the LSV is then dissecting from the surrounding tissue. While this procedure usually yields a usable LSV, the incision(s) is painful, is reported to suffer wound healing failures rates of up to 40% not infrequently mandating rehositalization and considerable expense and discomfort, is a possible source of infection, takes a long time to heal, and leaves a long, noticeable scar. Further, the harvested vein must be extensively handled in order to remove the surrounding tissue. That can result in damage to the LSV and possible early failure after the LSV is used as a graft.

In an attempt to solve the problems caused by removal of the ISV via a long incision or multiple incisions in the leg, various endoscopic techniques have been developed. These techniques involve the insertion of aa endoscopic camera into the leg near the LSV at the knee area. The area around the camera may be inflated with a gas such as carbon dioxide using a gas-release nozzle positioned in an endoscopic dissection tool inserted along with the endoscopic camera - the gas is usually injected through a separate device requiring a separate incision. The endoscopic dissection tool is used to dissect the fatty tissue around the LSV and vein branches by gently using the pointed tip of the endoscopic dissection tool, the carbon dioxide gas flow and additional endoscopic dissection tools. After separating the LSV and vein branches from the fatty tissue, the dissection tools are withdrawn and endoscopic clipper is used to clip the various branches. Once that is completed a cutting tool (typically cauterizing scissors) is inserted through the endoscope. The cutting tool is manipulated to divide and cauterize the clipped branches of the vein. As used herein, the term "divide" when used in relation to a tubular body member means to cut entirely through the tubular body member.

After the LSV is dissected and the branches are clipped and divided as described above, incisions to expose the vein are made through the skin at the distal and proximal ends of the leg. The vein is ligated in continuity and then divided with a pair of scissors. The dissected LSV is then pulled out of the body.

While these endoscopic procedures reduce scaming, pain, wound-related complications and risk of infection as compared to the previously described open incision method, the endoscopic technique is both difficult to learn and to use. An endoscopic procedure can also damage the vein due to over manipulation and potential mishandling of the endoscopic tools. Additionally, endoscopic equipment used in these procedures is expensive to buy and use.

DE-A-19817979 discloses a tissue separation device which is moveable along a guide rod to remove a tubular tissue hollow structure, wherein the device can be formed out of two superimposed rotation scalpels.

### Summary of the Invention

The present invention provides a cutting head for use with a cutting tool for removing a tubular body member from the body according to claim 1. Optional features of the cutting head as well as a cutting tool, device and apparatus comprising the cutting tool are defined in claims 2 to 32.

A method of using the cutting tool of claim 1 improves upon the prior art methods for removing a tubular body member from the body and generally comprises the steps of (1) creating openings in the body through which the tubular body member can be accessed at a proximal end and a distal end, (2) sufficiently straightening the tubular body member to utilize a cutting tool according to the invention, (3) using a cutting tool to dissect a section of body tissue (wherein the tubular body member is inside the dissected section) between the proximal end and the distal end, and (4) removing the dissected section of tissue including the tubular body member from the body. Once the body tissue is removed from the body, the tubular body member is dissected from the body tissue using any suitable method.

One embodiment of the present invention is a percutaneous harvesting device (PHD) for the harvesting of tubular body members from a body, the PHD including (1) an endovascular component (EVC) for passing inside of the tubular body member to be removed, and (2) a perivascular cutting tool (PVT) that is inserted over the EVC and is used to cut a length of body tissue that includes the tubular body member, wherein the PVT includes a cutting head of claim 1.

In one preferred embodiment, the EVC comprises a guide wire and an endovascular guide (EVG) surrounding the guide wire. The EVG is preferably a catheter made from a soft material (preferably plastic) suitable for passing through the selected tubular body member without damage to the intimal surface of the tubular body member. The EVG may have a tapered end, or nose, to assist in introducing it into the tubular body member and may include one or more structures; such as grooves or rings, for securing the tubular body member to the EVG. Alternatively, the tubular body member can be secured to a specially designed, nozzle nosed, torque device with an external structure (such as an annular ridge or chevron) on the torque device, preferably positioned at the base of the nozzle. The nozzle is designed to fit partially inside the lumen of the tubular body member and to retain the tubular body member, preferably by a suture at the external structure for ligating the tubular body member to the torque device. The torque device is then tightened onto the guide wire at the proximal end and the distal end, thus the tubular body member is firmly fixed to the guide wire via the torque device.

The PVT is preferably cylindrical and has a diameter (or width) greater than the diameter of the tubular body member. The PVT surrounds the tubular body member and cuts through the body tissue surrounding the tubular body member thus dissecting from the body an essentially cylindrical section of body tissue (mostly fat in the case of the LSV) with the tubular body member inside the body tissue. If the tubular body member is a blood vessel, the blade cuts the branches of the blood vessel, thus isolating the blood vessel and enabling it to be removed without tearing. The dissected section of body tissue is removed from the body and the tubular body member is separated from the surrounding tissue in the dissected section.

In one preferred embodiment, the PVT includes a cutting head and a body section The cutting head preferably has an annular leading edge that forms an annular cutting blade.

The body section of the PVT is preferably a plastic tube having an attachment structure (preferably threads) at one end for attaching to the cutting head. Optionally, the body section includes an outer surface (or exterior) having a helical thread or other device on the outer surface to assist in the movement of the cutting tool through the body.

The PVT optionally is used in conjunction with a handle that can attach to an end of the body section of the PVT. The handle, and hence the PVT, is preferably turned by a user, such as a surgeon, to advance the PVT through a body to dissect the tubular body member. The handle may be an elongated shaft with one end that is connected to the body section of the PVT. Optionally, a handgrip can be attached to the handle for easier operation. Optionally, the PVT can be fitted with a power source, such as a battery pack and appropriate drive equipment to rotate or vibrate the PVT thus assisting in the dissection of the tubular body member with less resistance.

Thus, embodiments comprising a PHD provide a less invasive and quicker way of removing tubular vessels such as the long sapheneous vein (LSV) from a body.

### Brief Description of the Drawings

Aspects of the present invention will be appreciated with reference to the description of the invention when made with reference to the accompanying drawings, which show embodiments of the invention as well as optional features that may be applied to the cutting head, cutting tool, device and apparatus of the present invention, and wherein:
FIG. 1 is a side view of a perivascular harvesting device showing features present in preferred embodiments;
FIG. 2 is a cross-sectional, side view of the endovascular component of the percutaneous harvesting device of FIG.1;
FIG. 3 is a cross-sectional, side view of an alternate endovascular component;
FIG. 3a is an end view of an alternate endovascular component;

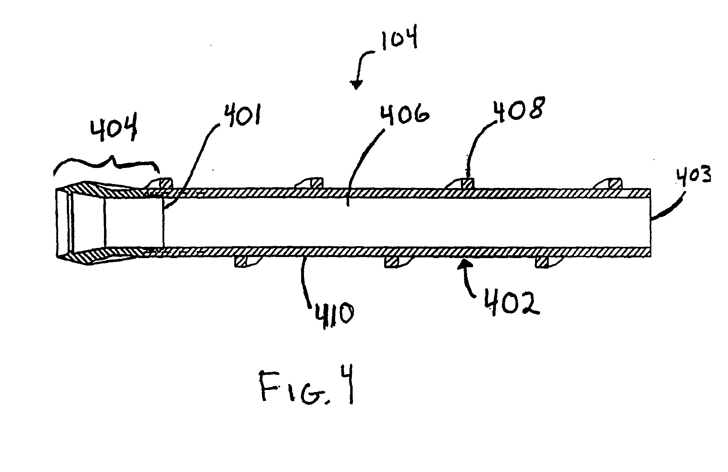

FIG. 4 is a cross sectional view of a perivascular cutting tool;
FIGS. 5a-5b are views of a cutting head for use with a perivascular cutting tool showing features present in preferred embodiments;
FIG. 6a is a side view of a perivascular cutting tool, being an embodiment of the present invention.
FIG. 6b is a cross-sectional, side view of the perivascular cutting tool shown in FIG. 6a;
FIGS. 7a-7b are views of an alternative cutting head for use with the perivascular cutting tool of FIGS. 6a-6b;
FIGS. 8a-8c are views of a connector for use with the cutting head of FIGS. 6a-6b and 7a-7b;

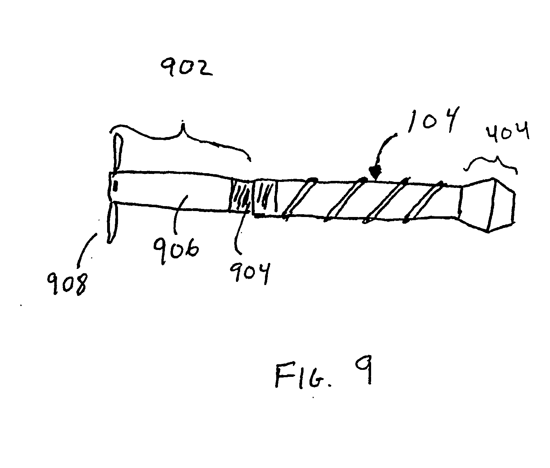

FIG. 9 is a view of a cutting tool that includes an optional handle and an optional hand grip,
FIG.10 is a view of a percutaneous harvesting device, being an embodiment of the present invention in use;
FIG.11 is a flow diagram of a method of using a device according to the invention.
FIG.12 is a view of a perivascular cutting tool, which has an automatic advancement device.

### Detailed Description of the Preferred Embodiments and examples illustrating preferred features

In the following descriptions, the present invention is frequently discussed using the example of the removal of a blood vessel, such as the LSV, from the body. However, the devices and methods of the invention can be used to harvest any tubular body member from a body for any purpose.

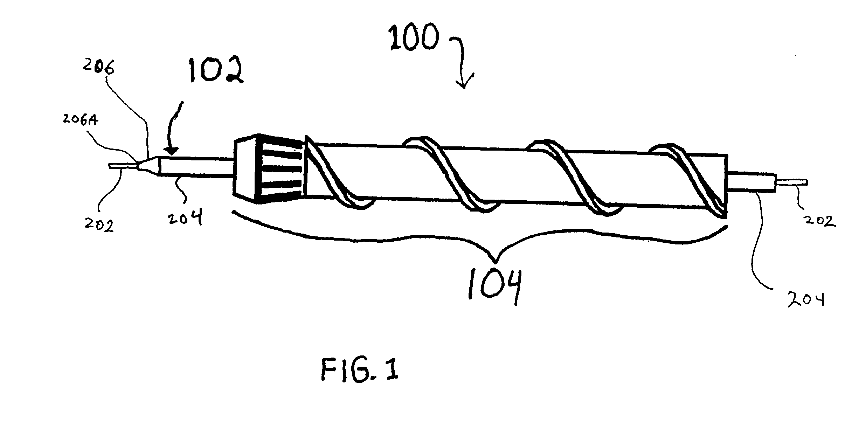

Turning now to the drawing figures where the purpose is to describe preferred embodiments and preferred features of the invention and not to limit same, a percutaneous harvesting device (PHD) 100 is illustrated in FIG. 1. PHD 100 is used percutaneously for dissecting a tubular body structure, such as a blood vessel, by cutting through a length of body tissue that includes the tubular body member, thus freeing the body tissue including the tubular body member from the body.

PHD 100 includes an endovascular component (EVC) 102 for insertion into the blood vessel to be removed, and a perivascular cutting tool (PCT) 104 for traveling subcutaneously and coaxially outside of and along the length of the blood vessel in which EVC 102 has been inserted. Cutting tool 104 is for cutting tissue surrounding the blood vessel.

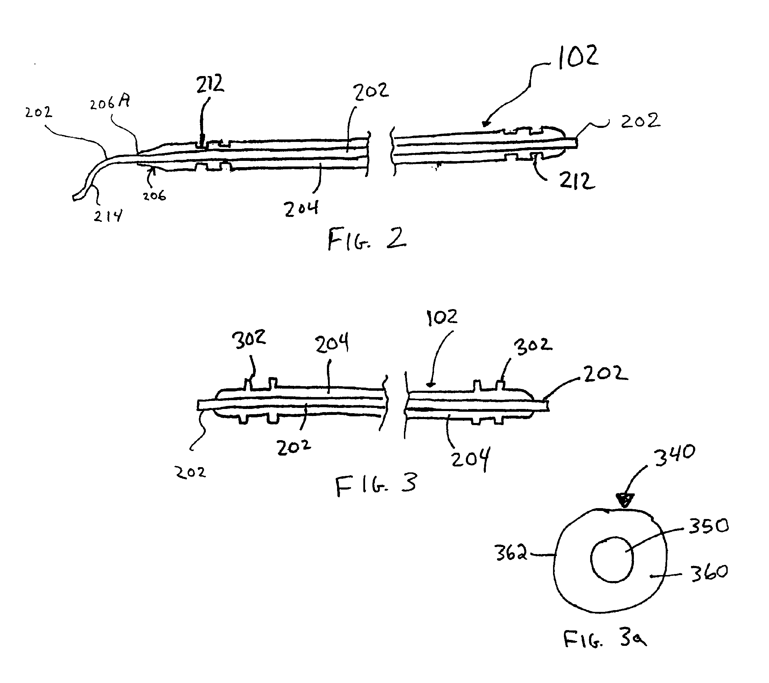

BVC 102 may be any structure or structures suitable for sufficiently straightening the blood vessel so that the blood vessel can be dissected from the body using a PVT according to the invention. Referring to FIG. 2, in one embodiment EVC 102 comprises a guide wire 202 and an outer tube, or endovascular guide (EVG), 204.surrounding guide wire 202, although it is possible that the EVC could be a single tubular member threaded through the vein. In the preferred method of using EVC 102, guide wire 202 is first inserted into the blood vessel and EVG 204 is then inserted over guide wire 202 and threaded through the blood vesseL

Guide wire 202 is any suitable medical guide wire that can be used in a procedure of using the cutting tool according to the invention, and guide wire 202 preferably has a hydrophilic coating and a straight floppy tip 214 to help provide maneuverability through the blood vessel. U.S. Provisional Application 60/475,666 to Opie and Joyce, filed on June 3, 2003 and entitled "Improved Medical Guide Wires" discusses exemplary guide wires. Suitable guide wires include those having a diameter between 0·025cm-0·097cm (.010"-.038") and having about a 2-5 cm long floppy tip. The guide wire is long enough to pass through and extend outside of each divided end of the blood vessel and is preferably about 40% longer than the section of blood vessel to be removed. If used to remove an LSV, the guide wire is usually about 230-260 cm in length.

EVG 204 is preferably a flexible tubular plastic catheter and includes a central lumen through which guide wire 202 is positioned when EVC 102 is positioned in the vein. EVG 204 optionally includes a tapered nose 206 that allows for easier introduction of EVG 204 into a blood vessel and easier passage through the blood vessel, and any structure suitable for this purpose may be used. Tapered nose 206 preferably is between about 3 and 4.5 cm in length and tapers to a tip 206A that is about 1.5 mm in diameter. EVG 204 can be soft enough to allow a suture to be secured to the EVG 204 in order to secure an end of a blood vessel thereto to facilitate removal Alternatively the suture can be applied to a guide wire torque device to secure the blood vessel as described herein. A guide wire torque device (or simply "wire torque device" or "torque device") is a device that mechanically grips and secures a guide wire. Suitable wire torque devices are disclosed in copending application Serial No.10/444,776, entitled "Guide Wire Torque Device" by Opie and Joyce.

Typically, EVG 204 is 10% to 20% longer than the section of blood vessel to be removed because both ends of EVG 102 need to be exposed outside of the respective divided ends of the blood vessel. The outside diameter of the EVG depends on the size of the blood vessel because the EVG must be of a suitable size to pass through the blood vessel without damaging it. In a preferred embodiment, the EVG has a diameter of 3-4 mm.

In one embodiment, a series of grooves 212 are formed around the circumference of each end of EVC 102. Grooves 212 are for securing the blood vessel to EVC 102, preferably at each end of EVC 102 and preferably through the use of suture ligatures. In this embodiment the grooves are preferably about 0.5 mm deep and are spaced about 1.0 centimeter apart.

In another embodiment, grooves 212 (shown in FIG. 2) may be replaced by a series of rings 302 (shown in FIG. 3). In this embodiment, rings 302 are about 2.0 mm wide and are spaced about 1.0 cm apart. The blood vessel to be removed can be attached to rings 302 via sutures or clips. In addition to grooves 212 and rings 302, any other structure that allows a blood vessel to be attached to EVC 102 may be used, or BVC 102 may not include any such structure. Any such structures for attaching a blood vessel to EVC 102, if used, can be positioned at any suitable location on the EVC. In two know embodiments such structures are located on the EVG about 25-40 cm from each end of an EVG 80-150 cm in length, and 65-80 cm from each end of an EVG 230 cm in length. Alternatively, these structures may be replaced by structures on torque devices positioned at either end of the EVG, wherein the tubular body member can be secured to the structure on each torque device.

Presently, the most preferred embodiment includes a EVC 102 having a guide wire with a hydrophilic coating and a single floppy tip and an EVG made of PVC or similar, suitable plastic, approximately 4 mm in diameter and having a central lumen of about 1-1.3 mm in diameter, wherein the EVG is threaded over the guide wire.

An alternate EVC 340 is shown in FIG. 3a. EVC 340 comprises an inner core 350 covered by an exterior covering 360. Inner core 350 provides both strength and flexibility to EVC 340, and is preferably made from a flexible or semi-flexible polymer plastic. However, any material that provides for a firm but semi-flexible inner core can be used. In one embodiment, the inner core is 2-3 mm in diameter. Any diameter can be used, however, as long as EVC 340 is properly sized to be threaded through the blood vessel to be removed.

The preferred exterior covering 360 is deformable and deforms in response to pressure from a suture or clip (such as a C-clip) in order to secure an end of a blood vessel to EVC 340 without significant slippage. The exterior covering can be made from foamed plastic, silastic or silicone rubber, although any suitable bio-compatible material can be used. In one embodiment, exterior covering 360 is 0.5 to 1.0 mm thick, although other thickness can be used with the maximum thickness controlled by the overall thickness of EVC 340, which needs to be properly sized to fit inside a blood vessel and is typically 3-4 mm in diameter if used in the removal of the LSV.

Exterior covering 360 may be co-extensive with inner core 350 or it may cover only part of an area of the inner core 350. In one embodiment the exterior covering 360 may cover from 25 to 40 cm from one end of inner core 350 for a shorter EVC 340, or 65-80 cm from one end of inner core 350 for a longer EVC 340. EVC 340 may also include a nose or cone having dimensions the same as or similar to those of previously described structure 206.

The PHD further includes a perivascular cutting tool (PVT) 104. PVT 104 includes a body section 402 coupled to a cutting head 404. As used herein, unless otherwise stated, "coupled" means attached in any manner suitable for the PVT to be used in the manner described herein.

Body section 402 is any suitable suitable for use in a method of removing a tubular body member from a body and is preferably a hollow tubular structure having a first end 401, a second end 403. a passage 406 extending therethrough and an optional driving helix 408 positioned on annular wall 410. Body section 402 is preferably an extruded, semi-flexible polycarbonate (such as General Electric Lexan 12) piece flexible enough to be suitable for the particular application in which it is to be used. Body section 402 supports cutting head 404 and preferably helps to substantially center the cutting head 404 around the blood vessel being removed during the cutting procedure. Body section 402, in one embodiment, has an exterior diameter of 15 mm and an internal diameter of 10 mm and is approximately 100 cm in length, and in this embodiment wall 410 is preferably about 3 mm thick. Body section 402 may have different dimensions, however, the dimensions depending upon such factors as the application for which the PVT will be used and the amount of surrounding tissue to be removed with the blood vessel. The outer surface of wall 410 is preferably coated with a low-friction material, such as TEFLON, to reduce friction during use, or may be coated with a hydrophilic coating such as polyurethane.

Driving helix 408 is optional and is preferably a 2mm high, clockwise, helical thread positioned on (i.e., formed in or attached to) the outer surface of wall 410 of body section 402. Helix 408 assists in advancing PCT 104 through the body, and any structure positioned on wall 410 suitable for performing this function may be used, assuming such a structure is used at all. For example, other sizes and types of threads may be used, or a series of longitudinally-extending grooves may be positioned on in the outer surface of wall 410, and the grooves may be slightly twisted to provide gripping ability.

As PVT 104 is turned, driving helix 408 grips the body tissue through which it is passing and helps to advance PVT 104 forward, and/or helps to prevent PVT 104 from slipping backward during a procedure. In one embodiment, driving helix 408 is dimensioned such that for every 360° rotation of body portion 402, PVT 104 would advance 3.0 centimeters if there were no slippage, with a preferred range of 2-3 cm of advancement for every 360° rotation of body portion 402.

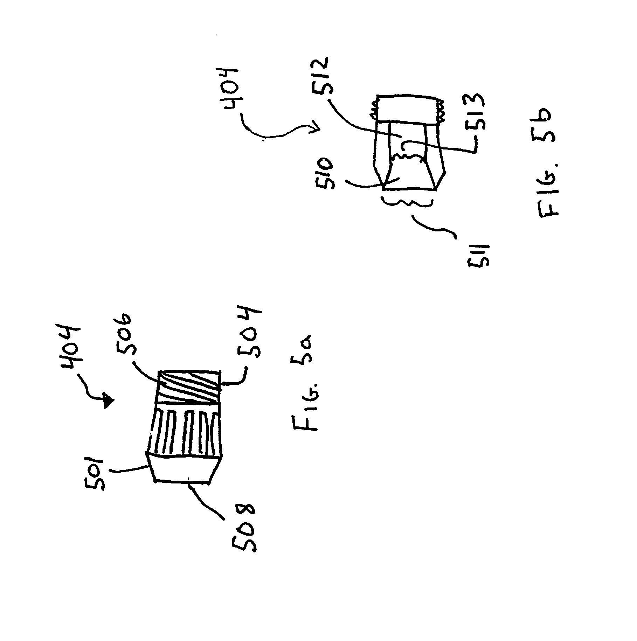

Cutting head 404 is designed to cut the body tissue surrounding the blood vessel and to cut blood vessel branches, thus dissecting the body tissue from the body so that the body tissue including the blood vessel can be removed. Cutting head 404 is preferably metal (most preferably carbon steel or stainless steel). Referring to FIGS. 5a and 5b, cutting head 404 has, in one embodiment, a generally wedge-shaped front 501 (as seen in side view), approximately in the shape of a truncated cone. The shape of front 501 assists in the movement of PVT 104 inside the body by pushing tissue outward from PVT 104 as cutting head 404 advances. While cutting head 404 with front 501 is illustrated, cutting head 404 can be any suitable shape for use on the PVT.

Cutting head 404 includes an attachment end 504. Attachment end 504, in one embodiment, has threads 506 that threadingly engage first one 401 of body portion 402, although cutting head 404 can be connected to body section 402 by any method or structure that provides a secure connection. Preferably, cutting head 404 is removable from body section 402, so that it may be disposed of (if desired), while body section 402 can be sterilized and reused (if desired).

Cutting head 404 includes a leading edge forming an annular blade 508. With annular blade 508 at the leading edge of PVT 104 the force required to advance PVT 104 through the body tissue is less than the force that would be required if the blade was behind the leading edge. Further, the annular blade provides 360 degrees of cutting surface, which also reduces the amount of force that must be applied to advance the PVT relative a cutting surface of less than 360°. Annular blade 508 is, in one embodiment, non-senated, although a serrated annular blade can also be used.

Cutting head 404 also includes, in one embodiment, and with reference to the cross-sectional view of FIG. 5b, an internal funel-shaped section 510 coupled to infernal cylindrical section 512. Internal funnel-shaped section 510 compresses tissue dissected by the cutting blade. In this respect, as PVT 104 advances, the dissected tissue is forced into section 510 by the forward movement of PVT 104. In section 510 the body tissue is compressed from a first diameter essentially equal to diameter 511, down to a second diameter essentially equal to diameter 513. The compression of the body time helps keep the PVT 104 essentially centered around the blood vessel to be removed, which helps to prevent the blood vessel from being cut by cutting blade 508.

In one embodiment first diameter 511, i.e., the diameter of annular blade 508, is 15mm and second diameter 513, i.e., the diameter of the internal cylindrical section 512, is 10mm. The length of internal funnel section 510 is, in one embodiment 10 mm, and is preferably in the range of 5 to 15 mm.

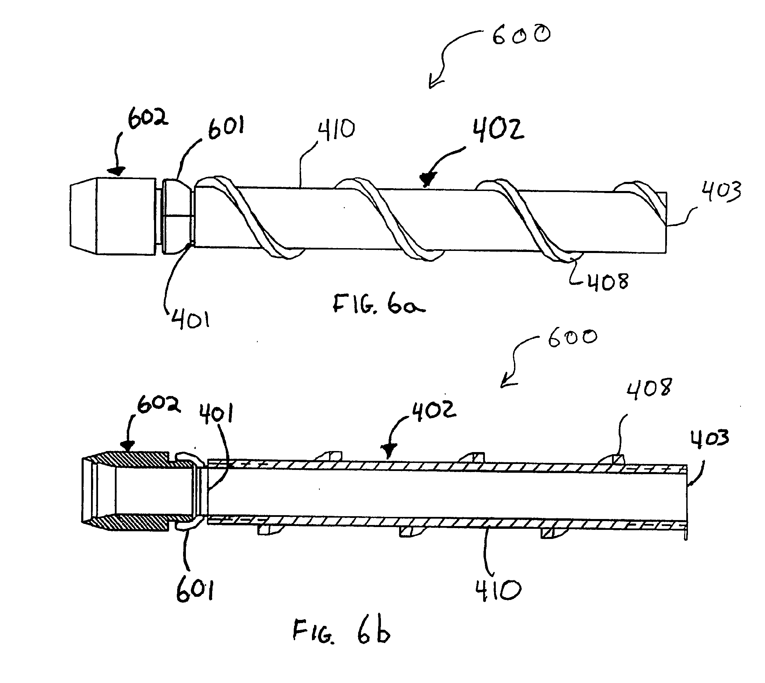

With reference to FIGS. 6a and 6b, an articulated PVT 600 is shown. Articulated PVT 600 has an articulated (i.e., jointed) cutting head 602 that is able to move and pivot independent from body portion 402 and any structure suitable for allowing cutting head 602 to pivot may be used. The articulation allows for easier movement of the articulated PVT 600 around structures such as the knee. In one embodiment, articulated cutting head 602 can pivot up to 15 degrees, although any suitable pivoting range may be utilized. Unless otherwise stated, the preferred size, shape, materials and configuration of cutting head 602 are the same as previously described for cutting head 404. Articulated PVT 600 also includes a body section 402 (previously described) and an articulated connection section 601. Articulated cutting head 602 is coupled to connection section 601, as best seen in FIG. 6b.

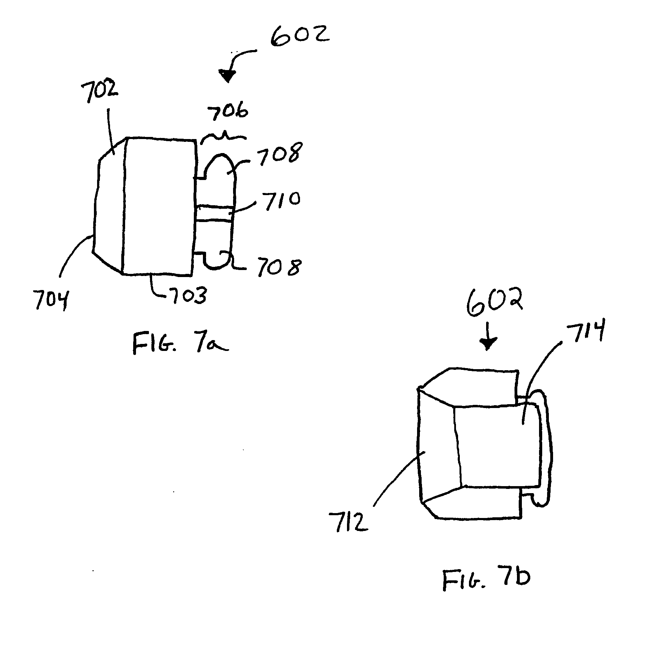

Referring to FIGS. 7a-7b, articulated cutting head 602 includes a blade portion 702 having a leading edge forming an annular cutting blade 704 and a coupling section 706. Coupling section 706 includes an annular rim 708 and a channel 710 formed in rim 708. Similar to cutting head 404, articulated cutting head 602 includes an internal funnel section 712 coupled to an internal cylindrical section 714. Internal funnel section 712 compresses tissue dissected by annular cutting blade 704. The advancement of articulated PVT 600 forces tissue to the cylindrical section 714. The compression of the body tissue helps keep articulated PVT 600 essentially centered around the blood vessel to be removed in order to assist in preventing the blood vessel from being cut by cutting blade 704.

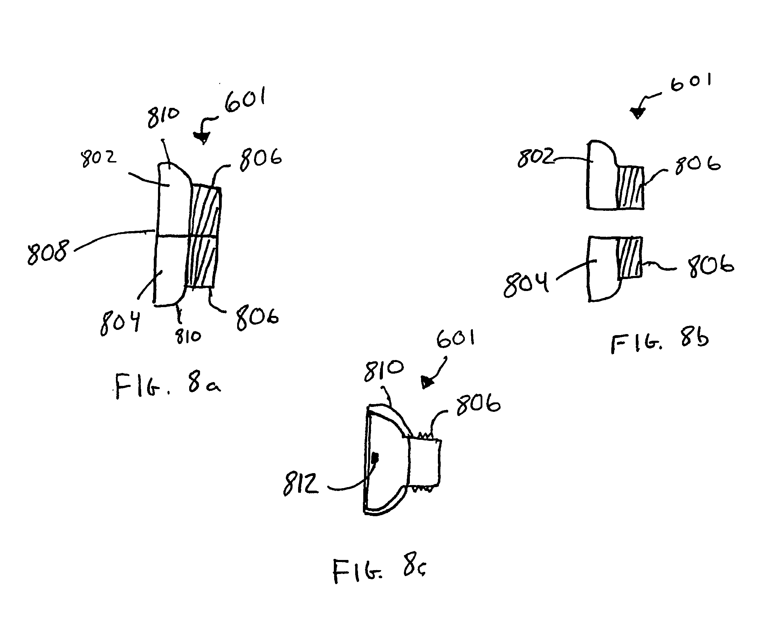

Referring to FIGS. 8a-8c, preferred connection section 601 comprises two parts, a first connection section 802 and a second connection section 804. Each connection section 802 and 804 has threads 806 and when connection sections 802 and 804 are joined, threads 806 form an essentially continuous thread that can be used to threadingly connect section 601 to body portion 402. Connection sections 802 and 804, when joined, form a cup 808 between connection sections 802 and 804.

Cup 808 includes a lip 810 that engages and retains annular rim 708 of articulated cutting head 602 and enables cutting head 602 to pivot. Each lip 810 includes a stud 812. When cup section 808 is coupled to coupling section 706, each stud 812 is aligned with and positioned inside of a channel section 710. In one embodiment there are two channel sections 710, each of which has a stud 812 positioned therein when cutting head 602 is coupled to connecting section 601. This prevents the articulated cutting head 602 from rotating continuously about a center axis, while still allowing the articulated cutting head 602 to pivot freely. If the cutting head 602 were allowed to continuously rotate, then the twisting motion that may be used to advance articulated PVT 600 inside the body could be translated into merely a spinning of articulated cutting head 602.

Once the articulated cutting head 602 is coupled to the articulated connection section 601, articulated cutting head 602 and articulated connection section 601 together act like a ball and socket joint wherein section 706 is analogous to the ball and cup section 808 is analogous to the socket. The articulation assists in the advancement of PVT 104 through the body. For example, articulated cutting head 602 allows PCT 600 to more easily maneuver around structures in the body, such as the knee joint.

Moving PVT 104 or 600 through the subcutaneous body tissue requires a certain amount of torque and/or pressure to force a PVT through the body tissue surrounding the blood vessel. To assist in advancing a PVT, an optional torque handle (or simply "handle") 902 can be attached to PVT 104 or 600 opposite the cutting head. Torque handle 902 is designed to fit into an adult human hand and is preferably a rod or tube made from extruded plastic (Lexan 12, high density polyethylene, acetal, Nylon, ABS are all plastics that could be used) and includes a connector 904 to connect to the PVT 104 and a shaft 906. In one embodiment connector 904 comprises threads on the rod or tube that threadingly connect to the PVT. Optionally a handgrip 908 can be attached to or formed in handle 902 to further assist in operation of the PVT.

A surgeon or other medical worked would first attach torque handle 902 to the PVT 104 or 600. This is done using connector 904, which connects to the end of PVT 104 or 600 opposite the cutting head, preferably by a threaded connection. Once connected and the PVT is inserted into the body, the surgeon twists and pushes the shaft 906. This causes the PVT to rotate and move forward. Cutting head 404 or 602 cuts the body tissue and the blood vessel and surrounding tissue is pressed through the body portion 402 as the PVT is advanced. Driving helix 408, if used, helps move the PVT forward and prevents the PVT from backing out of the body. To remove the PVT, the user would either advance it entirely out of the body or turn it in the opposite direction while pulling on the torque handle 902 to back it out of the body. Handgrip 908 can also be used to help in twisting the torque handle 902. Torque handle 902 and handgrip 908 can be made from a plastic such as polycarbonate, although any strong rigid material can be used.

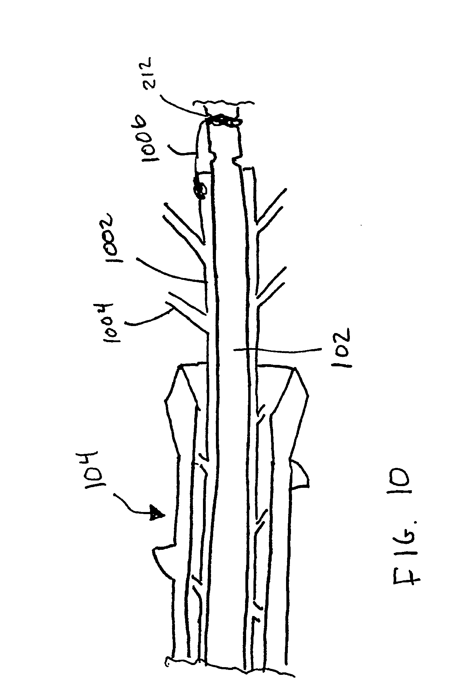

Referring now to FIG. 10, EVC 102 is shown positioned in a blood vessel 1002 to be removed. When EVC 102 is inserted into vessel 1002, blood vessel 1002 collapses around the endovascular guide 102 as the blood in blood vessel 1002 is pushed outward through branches 1004. As seen in FIG. 10, blood vessel 1002 can be secured to EVC 102 using a suture 1006 to secure to a structure 212 formed on EVG 204 or to the torque device as previously discussed. Alternatively, blood vessel 1002 may be secured to a wire torques device at one end or both ends. Typically, both ends of the blood vessel 1002 to be removed are secured to EVC 102 or to respective torque devices to help straighten blood vessel 1002 to be removed.

After endovascular guide 102 is inserted through blood vessel 1002, PVT 104 is passed along endovascular guide 102 such that endovascular guide 102 and blood vessel 1002 it is inserted into is inside PVT 104. As PVT 104 moves along endovascular guide 102 branches 1004 are severed by annular blade 508, which is on the leading edge of PVT 104. The diameter of annular blade 508 determines the length of branches 1004 left on removed blood vessel 1002. Cut blood vessel 1002 and surrounding tissue passes to body section 402.

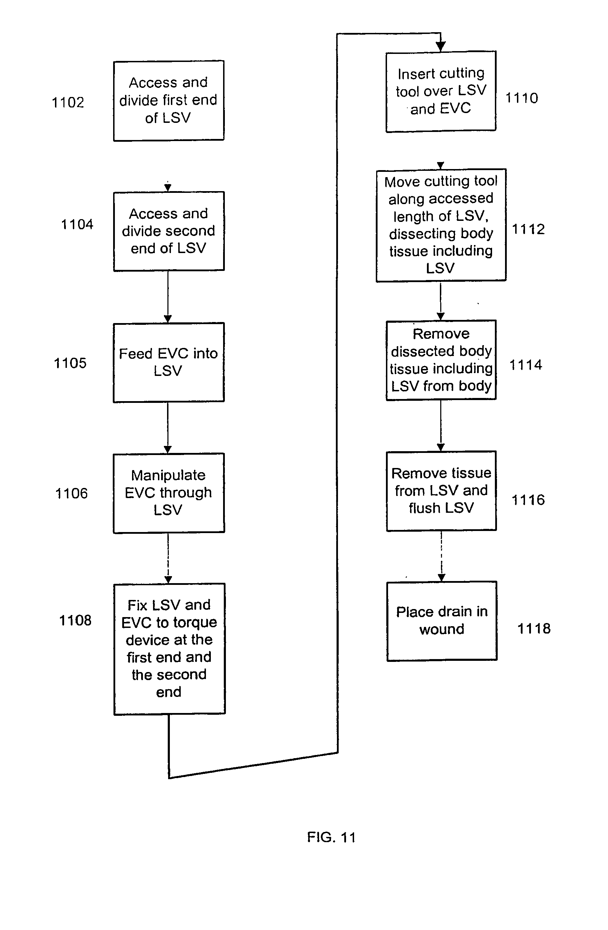

Referring to FIGS.10 and 11, a method of using a PHD as described herein shall be described. In this preferred harvesting (or removal) procedure, a PHD having either PVT 104 or PVT 600 may be utilized to remove an LSV. First, the LSV is accessed and divided at a proximal end (step 1102) and a distal end (step 1104). Next, a guide wire 202 is fed through the LSV and is exposed outside of the body at the proximal end and the distal end. EVG 204 is then advanced over guide wire 202 and into the LSV from the proximal end to the distal end and is exposed at each end (Steps 1105 and 1106).

To secure the guide wire a wire torque device (the wire torque devices are not shown) is preferably placed on the guide wire at the proximal end outside of the LSV and another guide wire torque device is placed on the guide wire at the distal end outside of the LSV. The LSV is secured at both the proximal end and the distal end to either the EVG or a wire torque device, and the EVG is secured to a wire torque device at the proximal end and to a wire torque device at the distal end. (Step 1108).

The guide wire, catheter and LSV are then pulled straight by applying force to the distal end and the proximal end of each, preferably by pulling on the wire torque devices. As used throughout this application with respect to straightening a tubular body member, the word "straight" means sufficiently straight to utilize a cutting tool according to the invention, and is not limited to a perfectly straight configuration.

Once the LSV is sufficiently straightened to remove it using a PVT as described herein, a PVT is utilized to dissect body tissue including the LSV from the body. The PVT is positioned so that the guide wire and EVG are inside the PVT and the LSV is preferably approximately axially-aligned with the cavity of the cutting head. (Step 1110). Ideally, the passage of the PVT is coaxially aligned with the LSV, although the alignment need not be coaxial, the LSV must simply be positioned so that it is not cut by the cutting blade.

The PVT is then advanced along the accessed length of the tubular body member, cutting through the body tissue surrounding the LSV and the LSV branches, thereby separating the body tissue and LSV from the body. (Step 1112). Once separated, the tissue including the LSV is removed from the body, which may be accomplished by simply by withdrawing the EVG with the LSV and surrounding tissue out of one of the incisions. (Step 1114). After being removed, the LSV is dissected from the surrounding body tissue and the vein can be flushed and the branches tied off. (Step 1116).

A drain, optionally positioned in the PVT, can be placed into the wound created by the PVT. The PVT is then removed leaving the drain in the leg precisely where the body tissue had been. (Step 1118). The drain would then be in place to remove blood and clots from the wound. Exemplary drains are disclosed in U.S. Provisional Application 60/476,663 filed on June 5, 2003 and entitled "Improved Surgical Drains," to Opie and Joyce.

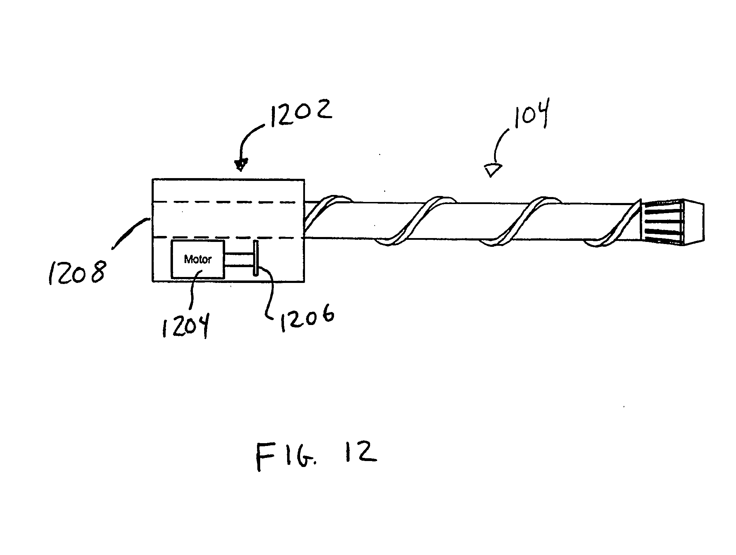

Manual operation of the PVT can be replaced or augmented by an electro-mechanical operation using an automated device. For example, and with reference to FIG. 12, the perivascular cutting tool 104 is coupled to an automatic advancement device 1202. Automatic advancement device 1202 applies a twisting motion, vibration or other suitable force to the PVT to assist in advancing the PVT through the body and may be any device suitable for this purpose. In one embodiment, automatic advancement device 1202 comprises a low speed, high torque motor 1204 that couples to either the PVT or to a torque handle. Motor 1204 would use gears 1206, belts or any other method of connecting a motor to a shaft to transfer driving force to the PVT. Preferably, automatic advancement device 1202 comprises a variable speed motor to vary the torque an/or force applied to the PVT to control the speed of the PVT. In one embodiment, automatic advancement device 1202 includes an opening 1208 for the passage of an endovascular guide wire.

In addition to, or as an alternative to, twisting PVT 104 or 600, automatic advancement device 1202 may also vibrate or otherwise manipulate PVT 104 or 600 to assist in moving it through the body. Such a movement could be provided, for example, by an ultrasonic vibrator.

Having now described preferred embodiments of the invention; modifications and variations that do not depart from the present invention may be made. The invention is thus not limited to the preferred embodiments, but is instead set forth in the following claims.

## Claims

1. A cutting head (602) for use with a cutting tool (600) for removing a tubular body member from a body, the cutting head comprising:
(a) a leading edge (702) comprising an annular cutting blade (704); and
(b) an attachment structure for attaching to a body section (402) of a cutting tool,
**characterised in that** the attachment structure allows the cutting head (602) to pivot when attached to the body section.

2. The cutting head (602) according to claim 1 further comprising an inner cavity extending therethrough, the inner cavity comprising a funnel-shaped section having a first diameter juxtaposed the leading edge and a second diameter, the second diameter being smaller than the first diameter.

3. The cutting head according to claim 1 wherein the cutting head further includes an inner cavity (712), the inner cavity being funnel-shaped and having a first inner diameter at the leading edge and a second inner diameter, the second inner diameter being smaller than the first inner diameter, the inner cavity compressing body tissue during operation of the cutting head to assist in keeping the tubular body member from being cut by the annular cutting blade.

4. The cutting head according to any one of claims 1 to 3 wherein the attachment structure comprises an annular rim (708) and an articulated connection section (601), the articulated connection section having a first end to engage and retain the angular rim and a second end for attaching to the body section.

5. The cutting head according to any one of claims 1 to 4 wherein the cutting head is comprised of steel.

6. The cutting head according to any one of claims 1 to 5 wherein the annular blade provides 360 degrees of cutting surface.

7. The cutting head according to any one of claims 1 to 6 wherein the annular blade is non-serrated.

8. A device for removing a tubular body member from a body, the device comprising a cutting tool (600) that includes:
(a) a cutting head (602) according to any one of claims 1 to 7; and
(b) a body section (402) having a proximal end, a distal end and an inner passage extending therethrough, the distal end operable to couple to the attachment structure of the cutting head.

9. The device according to claim 8 wherein the attachment structure of the cutting head is threaded and the distal end of the body section is threaded (806), the cutting head being attachable to the body section by threading it onto the distal end.

10. The device according to claim 8 or 9 wherein the body section is tubular.

11. The device according to any one of claims 8 to 10 wherein the body section has an exterior surface and the exterior surface is coated with a hydrophilic coating.

12. The device according to any one of claims 8 to 11 wherein the body section has an exterior surface and the exterior surface is coated with a low-friction coating.

13. The device according to any one of claims 8 to 12 wherein the body section further comprises an exterior surface, and a structure positioned on the exterior surface to assist in the movement of the cutting tool through body tissue.

14. The device according to claim 13 wherein the structure on the exterior surface is a helical thread (408).

15. The device according to any one of claims 8 to 14 further comprising an endovascular component (102) for being positioned in the tubular body member.

16. The device according to claim 15 wherein the endovascular component comprises a flexible tube (204) and a medical guide wire (202).

17. The device according to claim 15 or 16 wherein the endovascular component includes one or more structures to which the tubular body member can be attached.

18. The device according to any one of claims 8 to 17 wherein the body section is comprised of polycarbonate.

19. The device according to any one of claims 8 to 18 further comprising an automatic advancement device to assist in the movement of the device for removing a tubular body member through the body.

20. The device according to claim 19 wherein the automatic advancement device comprises an ultrasonic vibrator or electric motor.

21. The device according to any one of claims 8 to 20 further comprising a handle attached to the proximal end of the body section.

22. The device according to claim 21 wherein the handle comprises a cylindrical tube.

23. The device according to claim 21 or 22 further comprising a hand grip attached to the handle.

24. An apparatus for harvesting a tubular body member, the apparatus comprising:
(a) an endovascular component (102,340) having a diameter smaller than the diameter of the tubular body member, the endovascular component capable of being inserted into the tubular body member;
(b) a cutting tool (600) comprising:
(i) a tubular body section;
(ii) a cutting head (602) according to any one of claims 1 to 7 attached to the tubular body section; and
(iii) an opening extending through the cutting tool; wherein the opening is sized to allow the tubular body member and some body tissue surrounding the tubular body member to fit inside.

25. The apparatus according to claim 24 wherein the endovascular component includes an inner section and an outer section.

26. The apparatus according to claim 25 wherein the inner section comprises a medical guide wire.

27. The apparatus according to any one of claims 24 to 26 further comprising a torque handle (902) coupled to the tubular body section, the torque handle used to turn the apparatus to assist in moving it through the body.

28. The apparatus according to any one of claims 24 to 27 further comprising an automatic advancement device to assist in the movement of the apparatus through the body.

29. A device for removing a tubular body member from a body, the device comprising a cutting head according to anyone of claims 1 to 7.

30. The device according to claim 29 further comprising an exterior surface and a helical thread on the exterior surface.

31. A cutting tool (600) for removing a tubular body member from a body, the cutting tool comprising:
(a) a cutting head (602) according to anyone of claim 1 to 7, and
(b) a body section connectable to the cutting head, the body section having an exterior surface and a structure positioned on the exterior surface, the structure to assist in advancing the cutting tool through body tissue.

32. The cutting tool according to claim 31 wherein the structure is a helical thread (408).

## Patentansprüche

1. Schneidkopf (602) zur Verwendung mit einem Schneidinstrument (600) zur Entfernung eines röhrenförmigen Körperbestandteils aus einem Körper, wobei der Schneidkopf Folgendes umfasst:
(a) eine Vorderkante (702), die eine ringförmige Schneidklinge (704) umfasst, und
(b) eine Befestigungsstruktur zur Befestigung an einem Körperabschnitt (402) eines Schneidinstruments,
**dadurch gekennzeichnet, dass** die Befestigungsstruktur derart ausgebildet ist, dass der Schneidkopf (602) schwenkbar ist, wenn er an dem Körperabschnitt befestigt ist.

2. Schneidkopf (602) nach Anspruch 1, ferner umfassend einen inneren, sich durch diesen erstreckenden Hohlraum, wobei der innere Hohlraum einen trichterförmigen Abschnitt mit einem ersten Durchmesser, der an die Vorderkante angrenzt, und einem zweiten Durchmesser aufweist, wobei der zweite Durchmesser geringer ist als der erste Durchmesser.

3. Schneidkopf nach Anspruch 1, worin der Schneidkopf ferner einen inneren Hohlraum (712) umfasst, wobei der innere Hohlraum trichterförmig ist und einen ersten Durchmesser an der Vorderkante und einen zweiten Durchmesser aufweist, wobei der zweite Durchmesser kleiner ist als der erste Durchmesser, wobei der innere Hohlraum Körpergewebe während der Betätigung des Schneidkopfs zusammendrückt, um zu unterstützen, dass verhindert wird, dass der röhrenförmige Körperbestandteil durch die ringförmige Schneidklinge zerschnitten wird.

4. Schneidkopf nach einem der Ansprüche 1 bis 3, worin die Befestigungsstruktur einen ringförmigen Rand (708) und einen gelenkigen Verbindungsabschnitt (601) umfasst, wobei der gelenkige Verbindungsabschnitt ein erstes Ende, um mit dem ringförmigen Rand in Eingriff zu gelangen und diesen zu halten, und ein zweites Ende zur Befestigung an dem Körperabschnitt aufweist.

5. Schneidkopf nach einem der Ansprüche 1 bis 4, worin der Schneidkopf aus Stahl besteht.

6. Schneidkopf nach einem der Ansprüche 1 bis 5, worin die ringförmige Klinge eine 360°-Schneidfläche bereitstellt.

7. Schneidkopf nach einem der Ansprüche 1 bis 6, worin die ringförmige Klinge nicht gezähnt ist.

8. Vorrichtung zur Entfernung eines röhrenförmigen Körperbestandteils aus einem Körper, wobei die Vorrichtung ein Schneidinstrument (600) umfasst, das Folgendes umfasst:
(a) einen Schneidkopf (602) nach einem der Ansprüche 1 bis 7; und
(b) einen Körperabschnitt (402) mit einem proximalen Ende, einem distalen Ende und einem sich durch diesen hindurch erstreckenden inneren Durchlass, wobei das distale Ende betätigbar ist, um mit der Befestigungsstruktur des Schneidkopfes verbunden zu werden.

9. Vorrichtung nach Anspruch 8, worin die Befestigungsstruktur des Schneidkopfs ein Gewinde aufweist und das distale Ende des Körperabschnitts ebenfalls ein Gewinde (806) aufweist, wobei der Schneidkopf an dem Körperabschnitt befestigbar ist, indem er auf das distale Ende geschraubt wird.

10. Vorrichtung nach Anspruch 8 oder 9, worin der Körperabschnitt röhrenförmig ist.

11. Vorrichtung nach einem der Ansprüche 8 bis 10, worin der Körperabschnitt eine Außenoberfläche aufweist und die Außenoberfläche mit einer hydrophilen Beschichtung beschichtet ist.

12. Vorrichtung nach einem der Ansprüche 8 bis 11, worin der Körperabschnitt eine Außenoberfläche aufweist und die Außenoberfläche mit einer Beschichtung mit geringer Reibung beschichtet ist.

13. Vorrichtung nach einem der Ansprüche 8 bis 12, worin der Körperabschnitt ferner eine Außenoberfläche und eine auf der Außenoberfläche angeordnete Struktur zur Unterstützung der Bewegung des Schneidinstruments durch Körpergewebe umfasst.

14. Vorrichtung nach Anspruch 13, worin die Struktur auf der Außenoberfläche ein schraubenförmig gewundener Gewindegang (408) ist.

15. Vorrichtung nach einem der Ansprüche 8 bis 14, ferner umfassend eine endovaskuläre Komponente (102), welche in dem röhrenförmigen Körperbestandteil positionierbar ist.

16. Vorrichtung nach Anspruch 15, worin die endovaskuläre Komponente ein flexibles Röhrchen (204) und einen medizinischen Führungsdraht (202) umfasst.

17. Vorrichtung nach Anspruch 15 oder 16, worin die endovaskuläre Komponente eine oder mehrere Strukturen umfasst, an die der röhrenförmige Körperbestandteil befestigbar ist.

18. Vorrichtung nach einem der Ansprüche 8 bis 17, worin der Körperabschnitt aus Polycarbonat besteht.

19. Vorrichtung nach einem der Ansprüche 8 bis 18, ferner umfassend eine automatische Vorwärtsbewegungsvorrichtung zur Unterstützung der Bewegung der Vorrichtung zur Entfernung eines röhrenförmigen Körperbestandteils durch den Körper.

20. Vorrichtung nach Anspruch 19, worin die automatische Vorwärtsbewegungsvorrichtung einen Ultraschallschwingantrieb oder einen elektrischen Motor umfasst.

21. Vorrichtung nach einem der Ansprüche 8 bis 20, ferner umfassend einen an dem proximalen Ende des Körperabschnitts befestigten Griff.

22. Vorrichtung nach Anspruch 21, worin der Griff ein zylinderförmiges Rohr umfasst.

23. Vorrichtung nach Anspruch 21 oder 22, ferner umfassend einen an dem Griff befestigten Haltegriff.

24. Vorrichtung zur Gewinnung eines röhrenförmigen Körperbestandteils, wobei die Vorrichtung Folgendes umfasst:
(a) eine endovaskuläre Komponente (102, 340) mit einem geringeren Durchmesser als der Durchmesser des röhrenförmigen Körperbestandteils, wobei die endovaskuläre Komponente in den röhrenförmigen Körperbestandteil einführbar ist;
(b) ein Schneidinstrument (600), das Folgendes umfasst:
(i) einen röhrenförmigen Körperabschnitt;
(ii) einen Schneidkopf (602) nach einem der Ansprüche 1 bis 7, der an dem röhrenförmigen Körperabschnitt befestigt ist; und
(iii) eine Öffnung, die sich durch das Schneidinstrument erstreckt; worin die Öffnung so dimensioniert ist, dass sie zulässt, dass der röhrenförmige Körperbestandteil und etwas den röhrenförmigen Körperbestandteil umgebendes Körpergewebe hineinpasst.

25. Vorrichtung nach Anspruch 24, worin die endovaskuläre Komponente einen Innenabschnitt und einen Außenabschnitt umfasst.

26. Vorrichtung nach Anspruch 25, worin der Innenabschnitt einen medizinischen Führungsdraht umfasst.

27. Vorrichtung nach einem der Ansprüche 24 bis 26, ferner umfassend einen Drehgriff (902), der mit dem röhrenförmigen Körperabschnitt verbunden ist, wobei der Drehgriff eingesetzt wird, um die Vorrichtung zu drehen, um die Bewegung der Vorrichtung durch den Körper zu unterstützen.

28. Vorrichtung nach einem der Ansprüche 24 bis 27, ferner umfassend eine automatische Vorwärtsbewegungsvorrichtung zur Unterstützung der Bewegung der Vorrichtung durch den Körper.

29. Vorrichtung zur Entfernung eines röhrenförmigen Körperbestandteils aus einem Körper, wobei die Vorrichtung einen Schneidkopf nach einem der Ansprüche 1 bis 7 umfasst.

30. Vorrichtung nach Anspruch 29, ferner umfassend eine Außenoberfläche und einen schraubenförmig gewundenen Gewindegang auf der Außenoberfläche.

31. Schneidinstrument (600) zur Entfernung eines röhrenförmigen Körperbestandteils aus einem Körper, wobei das Schneidinstrument Folgendes umfasst:
(a) einen Schneidkopf (602) nach einem der Ansprüche 1 bis 7; und
(b) einen Körperabschnitt, der mit dem Schneidkopf verbindbar ist, wobei der Körperabschnitt eine Außenoberfläche und eine auf der Außenoberfläche angeordnete Struktur umfasst, wobei die Struktur dazu dient, die Vorwärtsbewegung des Schneidinstruments durch Körpergewebe zu unterstützen.

32. Schneidinstrument nach Anspruch 31, worin die Struktur ein schraubenförmig gewundener Gewindegang (408) ist.

## Revendications

1. Tête de coupe (602) pour utilisation avec un outil de coupe (600) pour retirer un élément de corps tubulaire d'un corps, la tête de coupe comprenant:
(a) un bord avant (702) comprenant une lame de coupe annulaire (704); et
(b) une structure de fixation pour la fixation à une section de corps (402) d'un outil de coupe, **caractérisée en ce que** la structure de fixation permet à la tête de coupe (602) de pivoter lorsqu'elle est fixée à la section de corps.

2. Tête de coupe selon la revendication 1, comprenant en outre une cavité interne s'étendant à travers celle-ci, la cavité interne comprenant une section en forme d'entonnoir ayant un premier diamètre juxtaposé au bord avant et un second diamètre, le second diamètre étant plus petit que le premier diamètre.

3. Tête de coupe selon la revendication 1, où la tête de coupe comprend en outre une cavité interne (712), la cavité interne étant en forme d'entonnoir et ayant un premier diamètre interne au bord avant et un second diamètre interne, le second diamètre interne étant plus petit que le premier diamètre interne, la cavité interne comprimant le tissu corporel durant l'opération de la tête de coupe pour contribuer à empêcher que l'élément de corps tubulaire soit coupé par la lame de coupe annulaire.

4. Tête de coupe selon l'une quelconque des revendications 1 à 3, où la structure de fixation comprend un bord annulaire (708) et une section de connection articulée (601), la section de connection articulée ayant une première extrémité destinée à venir en prise avec et retenir le bord annulaire et une seconde extrémité pour la fixation à la section de corps.

5. Tête de coupe selon l'une quelconque des revendications 1 à 4, où la tête de coupe est réalisée en acier.

6. Tête de coupe selon l'une quelconque des revendications 1 à 5, où la lame annulaire réalise 360 degrés de la surface de coupe.

7. Tête de coupe selon l'une quelconque des revendications 1 à 6, où la lame annulaire est non-dentelée.

8. Dispositif pour retirer un élément de corps tubulaire d'un corps, le dispositif comprenant un outil de coupe (600) qui comprend:
(a) une tête de coupe (602) selon l'une quelconque des revendications 1 à 7; et
(b) une section de corps (402) ayant une extrémité proximale, une extrémité distale et un passage interne s'étendant à travers celles-ci, l'extrémité distale étant opérable pour le couplage à la structure de fixation de la tête de coupe.

9. Dispositif selon la revendication 8, où la structure de fixation de la tête de coupe est filetée, et l'extrémité distale de la section de corps est filetée (806), la tête de coupe pouvant être fixée à la section de corps en la vissant sur l'extrémité distale.

10. Dispositif selon la revendication 8 ou 9, où la section de corps est tubulaire.

11. Dispositif selon l'une quelconque des revendications 8 à 10, où la section de corps présente une surface extérieure, et la surface extérieure est revêtue d'un revêtement hydrophile.

12. Dispositif selon l'une quelconque des revendications 8 à 11, où la section de corps présente une surface extérieure, et la surface extérieure est revêtue d'un revêtement basse friction.

13. Dispositif selon l'une quelconque des revendications 8 à 12, où la section de corps comprend en outre une surface extérieure, et une structure positionnée sur la surface extérieure pour contribuer au mouvement de l'outil de coupe à travers le tissu corporel.

14. Dispositif selon la revendication 13, où la structure sur la surface extérieure est un filet hélicoïdal (408).

15. Dispositif selon l'une quelconque des revendications 8 à 14, comprenant en outre un composant endovasculaire (102) destiné à être positionné dans l'élément de corps tubulaire.

16. Dispositif selon la revendication 15, où le composant endovasculaire comprend un tube flexible (204) et un fil de guidage médical (202).

17. Dispositif selon la revendication 15 ou 16, où le composant endovasculaire comprend une ou plusieurs structures auxquelles l'élément de corps tubulaire peut être fixé.

18. Dispositif selon l'une quelconque des revendications 8 à 17, où la section de corps est réalisée en polycarbonate.

19. Dispositif selon l'une quelconque des revendications 8 à 18, comprenant en outre un dispositif d'avancement automatique pour contribuer au mouvement du dispositif pour retirer un élément de corps tubulaire à travers le corps.

20. Dispositif selon la revendication 19, où le dispositif d'avancement automatique comprend un vibrateur ultrasonique ou moteur électrique.

21. Dispositif selon l'une quelconque des revendications 8 à 20, comprenant en outre une poignée fixée à l'extrémité proximale de la section de corps.

22. Dispositif selon la revendication 21, où la poignée comprend un tube cylindrique.

23. Dispositif selon la revendication 21 ou 22, comprenant en outre une prise fixée à la poignée.

24. Appareil pour le prélèvement d'un élément de corps tubulaire, l'appareil comprenant
(a) un composant endovasculaire (102, 840), ayant un diamètre plus petit que le diamètre de l'élément de corps tubulaire, le composant endosvasculaire pouvant être inséré dans l'élément de corps tubulaire;
(b) un outil de coupe (600) comprenant:
(i) une section de corps tubulaire;
(ii) une tête de coupe (602) selon l'une quelconque des revendications 1 à 7 fixée à la section de corps tubulaire; et
(iii)une ouverture s'étendant à travers l'outil de coupe; où l'ouverture est dimensionnée pour que l'élément de corps tubulaire et du tissu corporel entourant l'élément de corps tubulaire s'insèrent à l'intérieur.

25. Appareil selon la revendication 24, où le composant endovasculaire comprend une section interne et une section externe.

26. Appareil selon la revendication 25, où la section interne comprend un fil de guidage médical.

27. Appareil selon l'une quelconque des revendications 24 à 26, comprenant en outre une poignée de couple (902) couplée à la section de corps tubulaire, la poignée de couple étant utilisée pour faire tourner l'appareil pour l'aider à passer à travers le corps.

28. Appareil selon l'une quelconque des revendications 24 à 27, comprenant en outre un dispositif d'avancement automatique pour contribuer au passage de l'appareil à travers le corps.

29. Dispositif pour retirer un élément de corps tubulaire d'un corps, le dispositif comprenant une tête de coupe selon l'une quelconque des revendications 1 à 7.

30. Dispositif selon la revendication 29, comprenant en outre une surface extérieure et un filet hélicoïdal sur la surface extérieure.

31. Outil de coupe (600) pour retirer un élément de corps tubulaire d'un corps, l'outil de coupe comprenant:
(a) une tête de coupe (602) selon l'une quelconque des revendications 1 à 7; et
(b) une section de corps pouvant être reliée à la tête de coupe, la section de corps ayant une surface extérieure et une structure positionnée sur la surface extérieure, la structure pour contribuer à faire avancer l'outil de coupe à travers le tissu corporel.

32. Outil de coupe selon la revendication 31, où la structure est un filet hélicoïdal (408).
